(19) Europäisches Patentamt
European Patent Office
Office européen des brevets

(11) **EP 1 521 877 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**21.07.2010 Patentblatt 2010/29**

(45) Hinweis auf die Patenterteilung:
**14.12.2005 Patentblatt 2005/50**

(21) Anmeldenummer: **03763854.1**

(22) Anmeldetag: **15.07.2003**

(51) Int Cl.:
*D04H 1/46* (2006.01)    *D04H 1/56* (2006.01)

(86) Internationale Anmeldenummer:
**PCT/EP2003/007629**

(87) Internationale Veröffentlichungsnummer:
**WO 2004/007824 (22.01.2004 Gazette 2004/04)**

(54) **KOSMETISCHES WATTEPAD**

COSMETIC COTTON DISC

RONDELLE D'OUATE USAGE COSM TIQUE

(84) Benannte Vertragsstaaten:
**AT BE BG CH CY CZ DE DK EE ES FI FR GB GR HU IE IT LI LU MC NL PT RO SE SI SK TR**

(30) Priorität: **15.07.2002 EP 02400033**

(43) Veröffentlichungstag der Anmeldung:
**13.04.2005 Patentblatt 2005/15**

(73) Patentinhaber: **Paul Hartmann AG
89504 Heidenheim (DE)**

(72) Erfinder:
• **MANGOLD, Rainer
89542 Herbrechtingen (DE)**
• **RÖMPP, Angela
73119 Zell u.A. (DE)**
• **MICHELMANN, Jana
89522 Heidenheim (DE)**

(74) Vertreter: **Friz, Oliver et al
Dreiss Patentanwälte
Postfach 10 37 62
70032 Stuttgart (DE)**

(56) Entgegenhaltungen:
EP-A- 0 245 017    EP-A- 0 363 533
EP-A- 0 365 160    EP-A- 0 405 043
EP-A- 0 547 604    EP-A- 0 826 811
EP-A- 0 926 288    EP-A- 1 045 059
EP-A- 1 106 723    WO-A-94/11557
WO-A-99/24551    FR-A- 2 276 030
FR-A- 2 795 100    US-A- 3 837 995
US-A- 4 100 324    US-A- 4 559 157
US-A- 4 753 843

• **THE TEXTILE INSTITUTE: "TEXTILE TERMS AND DEFINITIONS" September 1988 (1988-09) , THE TEXTILE INSTITUTE , MANCHESTER XP002227582 Seite 239, Absatz 7**

EP 1 521 877 B2

**Beschreibung**

Die Erfindung betrifft ein kosmetisches Wattepad, d. h. ein Wattepad zum Reinigen der Haut oder zum Abschminken, aber auch zum Auftragen kosmetischer Produkte, wie Cremes, auf die Haut.

Kosmetische Wattepads sind vielfach bekannt geworden. So offenbart EP 1 106 723 Al ein Baumwoll-Wattepad mit beidseits wasserstrahlvernadelter Oberfläche. Das Baumwoll-Wattepad kann aber auch 0 - 30 % Kunstfasern, wie Viskosefasern, Polyesterfasern, Bikomponentenfasern, umfassen.

EP 0 836 842 B1 offenbart Wattepads und deren Herstellung, welche hydrophile Materialien umfassen, wie z. B. Baumwolle, Viskose oder Flachs, und/oder hydrophobe Materialien, wie z. B. Polyethylenterephtalat oder Polypropylen. Bevorzugt seien jedoch Wattepads, die zu 100 % aus Baumwolle bestehen oder solche, die zu 100 % aus Viskose bestehen oder aus wenigstens 25 % Viskose und der Rest Baumwolle oder aus wenigstens 25 % Baumwolle und der Rest Viskose oder aus 50 % Viskose und 50 % Polyethylenterephtalat oder aus 50 % Viskose und 50 % Polypropylen. Zur Faservliesverfestigung wird eine Wasserstrahlverfestigung vorgeschlagen.

EP 0 405 043 B1 offenbart ein dreischichtiges Wattepad, wobei die Schichten zu 100 % aus Baumwollkämmlingen bestehen. Die beiden äußeren Schichten sind stark verdichtet und an ihrer jeweiligen Sichtseite mit einem Waffelmuster versehen. Die drei Schichten werden dann zu einem sandwichartigen Aufbau zusammengeführt. Dabei sollen die Randpartien zumindest der äußeren Schichten miteinander verbunden werden.

Nach WO 00/76384 A1 soll ein Wattepad zu 100 % aus Baumwollfasern bestehen, wobei eine erste Schicht aus feinen Fasern einer Stärke von 0,7 - 1,75 denier (2 - 5 $\mu$g/pouce) und eine zweite Schicht aus demgegenüber stärkeren Fasern bestehen soll, welch letztere eine zum Abrieb bestimmte Oberfläche bilden soll.

US 4,100,324 beschreibt eine Fasermischung aus Holzzellstofffasern (Wood Pulp Fibers) und thermoplastischen Mikrofasern eines mittleren Faserdurchmessers von weniger als 10 $\mu$m. Dadurch dass diese Fasermischung quasi gleichzeitig mit dem Spinnen der Mikrofasern hergestellt wird, indem nämlich in den Bereich des Spinnkopfs für die Mikrofasern ein Luftstrom sowie ein Zellstoff-Faserstrom eingeströmt wird, entsteht eine Verbindung der Fasern und die Mikrofasern führen zu einer Fixierung der Zellstofffasern, und zwar in einem Zustand, in dem die Mikrofasern sich noch bei erhöhten Temperaturen in einem noch nicht gehärteten Zustand befinden. Die Mikrofasern wirken daher für die Holzzellstofffasern matrixbildend, wobei sie die Holzzellstofffasern gelenkig, also nicht starr einbinden, und zwar auch bei sehr geringen Gehalten an Mikrofasern bis herunter zu 1 Gew.-%. Es können zusätzlich weitere Fasern oder partikelförmiges Material, einschließlich synthetische Fasern wie Nylonfasern und natürliche Fasern wie Baumwolle, Flachs, Jute und Seide, eingebracht werden.

Durch die Herstellung der Fasermischung quasi in situ mit der Bildung der Mikrofasern im Schmelzblas-Prozess (melt blown-Verfahren) verlieren die Mikrofasern ihre Eigenständigkeit in dem Faserprodukt und werden aufgrund des Kontakts mit den Holzzellstoff-Fasern im noch erweichten Zustand verändert.

Der vorliegenden Erfindung liegt die Aufgabe zugrunde, ein kosmetisches Wattepad zu schaffen, welches sich einerseits sehr weich anfühlt, andererseits aber eine sehr hohe Reinigungs- oder Abschminkwirkung besitzt.

Diese Aufgabe wird erfindungsgemäß gelöst durch ein Wattepad mit den Merkmalen des Anspruchs 1.

Wenn vorstehend von Mikrostapelfasern die Rede ist, so werden hierunter synthetische Fasern einer Faserstärke von < 1 dtex verstanden. Der Begriff der Mikrostapelfasern bringt dabei zum Ausdruck, dass für die Herstellung der Faservlieslage des Wattepads zuvor in einem separaten Herstellungsverfahren gebildete Mikrofasern einer bestimmten Länge oder eines bestimmten Längenbereichs verwendet werden. Es hat sich gezeigt, dass synthetische Mikrostapelfasern einem Wattepad zu größerer Weichheit und damit zu einer hervorragenden Anfühlungswahrnehmung beim Benutzer zu verhelfen vermögen. Überraschenderweise ist damit aber nicht ein Rückgang der Reinigungs- oder Abschminkwirkung verbunden, sondern im Gegenteil weisen erfindungsgemäße Wattepads mit oder bestehend aus synthetischen Mikrostapelfasern einer Länge von wenigstens 7 mm, eine überlegene Reinigungs- und Abschminkwirkung auf.

Dies ist möglicherweise auf die aufgrund der Feinheit der Mikrostapelfasern zurückführbare große Oberfläche zurückzuführen, die zum Kontakt mit der zu reinigenden Hautoberfläche treten kann. Diese große Oberfläche begrenzt demzufolge auch eine riesige Anzahl von Mikrospalten und - öffnungen, welche zur Aufnahme von Unreinheiten, Hautschuppen oder Schminke dienen können.

Die Faserlänge der verwendbaren Mikrostapelfasern beträgt vorzugsweise 10 - 38 mm, insbesondere 15 - 32 mm.

Bei den Mikrostapelfasern kann es sich in weiterer Ausbildung der Erfindung um Polyester (PES) oder um Viskosefasern handeln. Die Oberfläche der Mikrostapelfasern sind vorzugsweise hydrophiliert. Auch kann die eine oder andere Oberfläche des Wattepads ein Prägemuster aufweisen. Solchenfalls kann das Prägemuster in an sich bekannter Weise, beispielsweise durch Kalanderprägung oder auch wie in EP-1106723-A2 oder WO-99/25318-A1 offenbart durch Wasserstrahlvernadelung erzeugt sein. Mit der Erzeugung des Prägemusters kann gleichzeitig eine Verfestigung des Vlieses einhergehen. Auch eine Vliesverfestigung durch den Zusatz wärmeschmelzbarer Bindefasern oder durch den Zusatz von chemischen Bindemitteln, wie z. B. wässrige Polymerdispersionen, z. B. Polyacrylate, Polyvinylacetate, Polyvinylalkohole, Latices oder Bindemittel auf Basis von Lösungsmitteln oder Polyurethane oder Streukleber/Schmelzklebepulver aus z. B. Polyamid, Polyethylen, Ethylenvinylacetat, Polyurethan oder Polyester, sind denkbar.

Das Flächengewicht des erfindungsgemäßen Kosmetikpads liegt vorzugsweise bei 120 - 250 g/m² und besonders bevorzugtermaßen bei 150 - 250 g/m².

Insbesondere wenn das kosmetische Wattepad zur Flüssigkeitsaufnahme ausgebildet werden soll, wenn es sich also bspw. gut zur Aufnahme einer gesichtsreinigenden Lösung eignen soll oder zum Abschminken unter Verwendung eines einen hohen Flüssigkeitsanteil aufweisenden Abschminkmittels, erweist es sich als vorteilhaft, wenn das Wattepad zusätzlich bis 72 Gew.-% Baumwollfasern, insbesondere 15 - 65 Gew.-% und weiter insbesondere 50 - 65 Gew.-% Baumwollfasern enthält. Hierbei handelt es sich vorteilhafterweise um Baumwollkämmlinge. Das Wattepad kann solche Baumwollfasern umfassen, die zu wenigstens 0,2 Gew.-% mit einem Weichmacher beaufschlagt sind. Dieser Weichmacher kann ein Fettsäurekondensationsprodukt und/oder funktionelle Polydimethylsiloxane und/oder Polyethylene umfassen.

Des Weiteren erweist es sich als besonders vorteilhaft im Hinblick auf die Erreichung einer hohen inneren Festigkeit bei dem erfindungsgemäßen Wattepad, wenn zusätzlich wärmeschmelzbare Bindefasern, vorzugsweise zu 10 - 20 Gew.-%, enthalten sind, und mit diesen Bindefasern eine thermische Verfestigung des Wattepads durchgeführt wurde. Der Anteil wärmeschmelzbarer Bindefasern beträgt insbesondere 12 - 18 Gew.-% und weiter insbesondere 12 - 15 Gew.-% bezogen auf die Masse des Wattepads.

In weiterer Ausbildung der Erfindung kann es sich bei den Bindefasern um Mehrkomponentenfasern, insbesondere Bikomponenten-Fasern handeln mit einer höher schmelzenden Trägerkomponente und einer niedriger schmelzenden Komponente.

Die Mehrkomponentenfasern, insbesondere Bikomponentenfasern, haben in vorteilhafter Weise eine Faserstärke von 1,3 - 10 dtex, insbesondere von 1,3 - 3 dtex und eine Faserlänge von 3 bis 60 mm. Vorteilhafterweise kommen Kern/Mantel-Fasern oder Seite-an-Seite-Fasern zum Einsatz.

Es hat sich als vorteilhaft erwiesen, wenn Bikomponentenfasern mit einem Copolyester (CO-PES) als niedrig schmelzender Komponente und Polyester (PES) als höher schmelzender Komponente verwendet werden.

In weiterer Ausbildung der Erfindung von besonderer Bedeutung ist der Schmelzpunkt der wärmeschmelzbaren Bindefasern oder der niedrig schmelzenden Komponente (z.B. CO-PES) der Mehrkomponentenfasern geringer als der Schmelzpunkt der Mikrostapelfasern. Bspw. könnten Mikrostapelfasern aus einem Polyestermaterial verwendet werden mit einem Schmelzpunkt von etwa 256°C und CO-PES/PES-Bikomponentenfasern mit einem Schmelzpunkt der niedrig schmelzenden Komponente CO-PES von 110°C und der höher schmelzenden Komponente PES von 255°C. Solchenfalls könnte eine thermische Verfestigung des Faservlieses durchgeführt werden, ohne die höher schmelzende Komponente der Bikomponentenfasern und die Mikrostapelfasern thermisch zu verändern.

Ferner wird in Weiterbildung der Erfindung vorgeschlagen, dass eine Seite des Wattepads oberflächenrau ausgebildet ist, indem dort ein Schleifmittel in Form einer Beschichtung aufgebracht ist. Somit wird ein Wattepad geschaffen, welches eine als sehr weich empfundene Seite aufweist, die eine, wie vorausgehend erläutert, sehr hohe Reinigungswirkung besitzt, und eine raue Seite, die zum "Peeling", also zum Entfernen von abgestorbenen Hautschuppen, verwendet werden. Gleichzeitig kann hierbei ein angenehmer Massageeffekt erzielt werden.

Das Schleifmittel kann in vorteilhafter Weise aus einem thermoplastischen Schmelzklebepulver gebildet sein. Das Schmelzklebepulver kann als pulverförmige Komponente, bspw. mittels eines Präzisionsstreuers über ein Vibrationssieb auf die eine Seite des Wattepads aufgebracht und dort thermisch fixiert werden. Die thermische Fixierung erfolgt vorteilhafterweise durch einen Sinterprozess in einem Ofen. Hierbei wird das Schmelzklebepulver nicht aufgeschmolzen, sondern die Partikel werden unter Beibehaltung der die oberflächenraue Struktur bildenden Partikelform mit dem Wattepad durch einen Sinterprozess verbunden. Das hierfür vorteilhafterweise zu verwendete Schmelzklebepulver umfasst Polyethylen und/oder Polyamid und/oder Polyester. Es weist eine Körnung von 1 - 500 μm, insbesondere von 1 - 100 μm, vorzugsweise von 1 - 65 μm auf. Es hat sich dabei als zweckmäßig und vorteilhaft erwiesen, wenn das Schleifmittel mit einem Flächengewicht von 5 - 50 g/m², insbesondere von 10 - 40 m² und vorzugsweise von 15 - 30 g/m² aufgebracht ist.

In weiterer Ausbildung des Erfindungsgedankens kann das erfindungsgemäße Wattepad herstellerseitig befeuchtet und im feuchten Zustand im Wesentlichen feuchtigkeitsdicht verpackt sein und dem Endverbraucher in dieser Form angeboten werden. Auch Feuchttücher, also Feuchttuchvliese, aus dem vorausgehend erörterten erfindungsgemäßen Faservliesmaterial sind von der Erfindung erfasst. Auch feuchte und trockene Wattestäbchen können hieraus hergestellt werden.

Auch feuchte Wattepads oder Feuchttücher umfassen vorzugsweise Baumwollfasern, die zu wenigstens 0,2 Gew.-% mit einem Weichmacher, insbesondere der vorausgehend beschriebenen Art beaufschlagt sind oder sein können. Vorteilhafterweise sind Feuchtpads mit einer Reinigungsemulsion in Form einer Öl-in-Wasser-Emulsion mit einer Viskosität von < 2000 mPas vorzugsweise von < 800 mPas befeuchtet. Die Öl-in-Wasser-Emulsion kann in einem Anteil von 0,2 - 0,5 Gew.-% einen Konservierungsstoff, bspw. Parabene oder Benzylalkohole, umfassen. Des Weiteren können 2 - 5 Gew.-% Pflanzenextraktanteil und 0,2 - 0,5 Gew.-% Parfumölanteil in der Öl-in-Wasser-Emulsion enthalten sein.

Die insbesondere feuchten Wattepads oder Feuchttücher weisen vorzugsweise eine geringe Lichtdurchlässigkeit von weniger als 0,9 %, vorzugsweise von weniger als 0,75 %, besonders bevorzugt von weniger als 0,70 %, ganz besonders bevorzugt von weniger als 0,6 % auf. Die Lichtdurchlässigkeit wird gemessen bei einer Wellenlänge von 600 nm mit einem Perkin-Elmer Lambda 2 UV/VIS Spektrophotometer an 30 mm x 40 mm großen Prüflingen.

Die Lichtundurchlässigkeit ist ein Hinweis auf die Packungsdichte des Fasermaterials und damit ein Maß für die zur Reinigungswirkung zur Verfügung stehende Faseroberfläche.

Es wurde in einem selbständigen Erindungsgedanken festgestellt, dass durch die Bestimmung der Lichtdurchlässigkeit eines insbesondere feuchten Wattepads oder eines Feuchttuchs zugleich die Reinigungswirkung dieses Wattepads oder Feuchttuchs festgestellt werden kann und somit die Reinigungswirkung verschiedener Wattepads oder Feuchttücher miteinander verglichen werden kann. Es wird daher selbständig Schutz beansprucht für ein insbesondere feuchtes Wattepad oder Feuchttuch auf Faserbasis mit einer geringen Lichtdurchlässigkeit von weniger als 0,9 %, vorzugsweise von weniger als 0,75 %, besonders bevorzugt von weniger als 0,70 %, ganz besonders bevorzugt von weniger als 0,6 %, und zwar auch unabhängig von der Art der verwandten Fasern und der Faserzusammensetzung.

Auf dem Markt verfügbare Feuchttücher weisen dagegen Lichtdurchlässigkeiten von 1,76 % (Nivea Visage, Erfrischende Reinigungstücher für normale Haut und Mischhaut, Beiersdorf AG), 2,26 % (Bebe Young Care Quick'n Clean für alle Hauttypen, Johnson & Johnson), 2,57 % (Diadermine, Pflegende Reinigungstücher für Gesicht und Augen, für alle Hauttypen, Schwarzkopf und Henkel Laboratoires Diadermine), 0,96 % (Demak´up Reinigungstücher, 100 % Baumwolle, für normale und Mischhaut, Georgia Pacific) auf.

In einer bevorzugten Ausführungsform der erfindungsgemäßen Feuchttücher wiesen die mit Emulsion versehenen Tücher eine Lichtdurchlässigkeit von 0,55 % (Feuchttuch 1) bzw. 0,48 % (Feuchttuch 2) auf. Die Zusammensetzung der Feuchttücher war dabei wie folgt:

Feuchttuch 1: 37% PES Mikrofasern mit einer Faserstärke von 0,9 dtex und einer Faserlänge von 18 mm , 23 % Baumwollkämmlinge, 40 % Viskosefasern mit einer Faserstärke von 1,7 dtex und einer Faserlänge von 40 mm. Das Flächengewicht des trockenen Faservlieses betrug 63 $g/m^2$. Die Dicke des trockenen Faservlieses betrug 0,66 mm, gemessen bei einem Druck von 0,5 kPa, wobei eine Prüffläche von 25 $cm^2$ zur Anwendung kam.

Feuchttuch 2: 49 % PES Mikrofasern mit einer Faserstärke von 0,9 dtex und einer Faserlänge von 18 mm , 51 % Baumwollkämmlinge. Das Flächengewicht des trockenen Faservlieses betrug 78 $g/m^2$. Die Dicke des trockenen Faservlieses betrug 0,80 mm, gemessen bei einem Druck von 0,5 kPa, wobei eine Prüffläche von 25 $cm^2$ zur Anwendung kam.

Feuchttuch 1 und 2 waren imprägniert mit Emulgade CM der Fa. Cognis Deutschland GmbH & Co.KG (PIT - Emulsion), einer Öl in Wasser - Emulsion bestehend aus Wasser, Cetearyl Isononanoat, Ceteareth 20, Cetearyl Alcohol, Phenoxyethanol, Parabene, Kaliumsorbat, Glycerin, Parfum, Zitronensäure, Cetyl Palmitat und Ceteareth 12 (INCI - Deklaration). Die Emulsionskonzentration betrug 7,5 %. Die Menge an Emulsion betrug 300% bezogen auf das trockene Gewicht des Faservlieses (d.h. auf 100g Vlies kommen 300g verdünnte Emulsion).

Zur Herstellung des erfindungsgemäßen Wattepads werden die in einem separaten Prozess hergestellten synthetischen Mikrostapelfasern zur Vliesbildung nach an sich üblichen Vliesbildungsverfahren abgelegt. Werden verschiedene Faserarten verwandt, so werden diese zuvor in einem Luftstrom vermischt und dann abgelegt. Sind wärmeschmelzbare Bindefasern vorhanden, so kann in einem "Air-through-Verfahren" mit genau einstellbarer Gastemperatur eine Thermofixierung des Vlieses vorgenommen werden, vorzugsweise ohne dass dabei die synthetischen Mikrostapelfasern thermisch und damit strukturell beeinflusst oder beeinträchtigt werden. Alternativ oder auch zusätzlich hierzu kann eine Wasserstrahlvernadelung der Faservliesbahn vor oder nach der Thermofixierung vorgesehen werden. Unabhängig hiervon ist eine einseitige oder zweiseitige Prägekalandrierung möglich, wobei das Wattepad mit einem Oberflächenmuster ausgestattet werden kann.

Das erfindungsgemäße Wattepad ist derart verdichtet, dass es eine Längsfestigkeit bzw. Höchstzugkraft in Längsrichtung (Maschinenrichtung) aufweist von 5 - 30 N/25mm, insbesondere 10 - 25 N/25 mm und vorzugsweise > 15 N/25 mm und eine Höchstzugkraft in Querrichtung (quer zur Maschinenrichtung) von 5 - 30 N/25mm, insbesondere 8 - 20 N/25mm und vorzugsweise > 10 N/25mm auf. Im Falle der Ausführungsform als Feuchttuch ist insbesondere eine Längsfestigkeit bzw. Höchstzugkraft in Längsrichtung des feuchten Tuches (Maschinenrichtung) von 5 - 70 N/25mm, ganz besonders von 10 - 60 N/25mm und vorzugsweise 30 - 50 N/25mm vorteilhaft und eine Höchstzugkraft in Querrichtung (quer zur Maschinenrichtung) von 5 - 60 N/25mm, insbesondere 10 - 50 N/25mm und vorzugsweise von 20-40 N/25mm besonders vorteilhaft. Diese Höchstzugkraft kann unter Verwendung einer genormten Zugprüfmaschine nach DIN 51221 nach der folgenden Prüfmethode ermittelt werden: Es werden aus dem zu prüfenden Wattepad, und zwar aus einem mittleren Bereich, Proben einer Einspannbreite von 25 mm und einer Einspannlänge von 30 mm genommen. Die in Klemmaufnahmen der normierten Zugprüfmaschine lotrecht eingespannten Proben werden dann mit einer Prüfgeschwindigkeit von 100 mm/min in der Ebene Ihrer Erstreckung auseinanderbewegt und dabei wird die in dieser Richtung wirkende Zugkraft gemessen. Unter der Höchstzugkraft wird diejenige maximale Kraft verstanden, bei der das Wattepad zerreist. Wenn zuvor höhere Kraftspitzen im Zuge der Dehnung gemessen werden, so stellen diese die Höchstzugkraft im Sinne dieser Prüfung dar. Man kann vorteilhafterweise bei Messungen der Längs- und der Querrichtung, welche der Maschinenrichtung bzw. der Richtung quer hierzu entspricht, verschiedene, insbesondere fünf Einzelmessungen vornehmen und deren Mittelwert berechnen.

Das erfindungsgemäße Wattepad weist ferner eine Dicke von vorzugsweise 0,5 - 4,5 mm auf, die unter einem spezifischen Messdruck von 0,5 kPa auf einer Tasterfläche von 25 cm$^2$ einer Probe des Prüflings ermittelt wurde. Das Prüfverfahren entspricht DIN EN ISO 9073-2 (Prüfverfahren für Vliesstoffe, Bestimmung der Dicke).

Des Weiteren kann das Absorptionsvermögen erfindungsgemäßer Wattepads bestimmt werden, und zwar wird hierfür entsprechend PH.EUR.1997, Monografie Verbandwatte aus Baumwolle, ein Test der Saugfähigkeit unter Bestimmung der Absinkdauer eines mit zu testenden Prüflingen befüllten Drahtkörbchens in einer Flüssigkeit ermittelt. Das hierzu verwendbare Drahtkörbchen ist ein zylindrischer Korb aus Kupferdraht mit einem Drahtdurchmesser von 0,4 mm. Die Höhe beträgt 80 mm, der Durchmesser 50 mm, die Maschenweite 15 - 20 mm und die Masse 2,7 +/- 0,3 g. Ferner findet ein Becherglas mit Durchmesser 11 - 12 cm Verwendung.

Zu testende Wattepads werden, bis eine Prüfmenge von 5 g vorhanden ist, in das Drahtkörbchen eingelegt. Zuvor wurde der Korb auf 0,01 g genau gewogen (M1). Die 5 g Probenmaterial bilden die Masse M2. Das Becherglas wird mit demineralisiertem Wasser bis zu einer Höhe von etwa 100 mm gefüllt und der gefüllte Korb wird aus einer Höhe von 10 mm auf das Wasser fallen gelassen. Mit einer Stopuhr wird die Zeit bis zum Absinken unter die Wasseroberfläche bestimmt. Unmittelbar nach der Bestimmung der Absinkdauer wird der Korb aus dem Wasser gehoben und zum Abtropfen 30 s lang in seiner Längsachse horizontal gehalten. Nach Ablauf der Abtropfzeit wird der Korb in ein tariertes Becherglas (M3) gegeben und auf 0,01 g genau gewogen (M4).

Das Wasserhaltevermögen ergibt sich

$$\text{in } g/g = \frac{M4 - (M2 + M3)}{M2 - M1}$$

Die Absinkdauer und das Wasserhaltevermögen werden als Mittelwert aus drei Bestimmungen angegeben. Für bevorzugte Wattepads beträgt die Absinkdauer maximal 15 sec und das Wasserhaltevermögen beträgt wenigstens 10 g/g. Dies lässt sich durch den Anteil absorbierender Fasern und/oder durch Zusatz von Hydrophilierungsmittel erreichen.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:

Figuren 1 - 8      das Ergebnis eines Versuchs zur Bestimmung der Reinigungs- bzw. Abschminkwirkung bei Wattepads,

Figuren 9, 10      zwei Oberflächenmuster von Wattepads,

Figur 11      das Oberflächenmuster nach Fig. 10 im Detail.

Zwei bevorzugte Zusammensetzungen des erfindungsgemäßen Wattepads sind nachfolgend angegeben; sie sind mit PH und PH* gekennzeichnet.

| Faserart | PH | PH* |
|---|---|---|
| Mikrostapelfasern PES | 25 Gew.-% | 47 Gew.-% |
| Baumwollkämmlinge | 62 Gew.-% | 40 Gew.-% |
| Bikomponentenfasern CO-PES/PES | 13 Gew.-% | 13 Gew.-% |

Die Wattepads PH und PH* wurden dadurch erhalten, dass die zuvor hergestellten Mikrostapelfasern einer Faserstärke von 0,9 dtex und einer Faserlänge von ca. 18 mm mit Baumwollkämmlingen und den Bikomponentenfasern in einem Luftstrom in der jeweiligen gewichtsprozentualen Zusammensetzung gemischt wurden. Bei den Mikrostapelfasern handelt es sich um Fasern aus einem Polyester mit einem Schmelzpunkt von 256°C, und bei den Bikomponentenfasern handelt es sich um Kernmantelfasern mit einer höher schmelzenden Kernkomponente aus einem Polyester mit einem Schmelzpunkt bei 255°C und einem Mantel aus einem Copolyester (CO-PES) mit Schmelzpunkt bei 110°C.

Die Länge dieser Bikomponentenfasern beträgt 51 mm; ihre Stärke 1,35 dtex.

Diese Wattepads wurden mit Vergleichsprodukten verglichen, wobei die nachfolgende Prüfung der Reinigungs- und Abschminkwirkung durchgeführt wurde: Hierbei wurde auf ein Substrat ein Make-up aufgebracht und unter standardisierten Bedingungen ermittelt, wie die jeweiligen Wattepads das Make-up von dem Substrat zu entfernen vermögen. Hierzu wird eine Zugprüfmaschine nach DIN 51221 Klasse 1 verwandt. Es wurde als Substrat "Ziegenglattleder beige zur Herstellung von Schuhen" verwandt. Auf ein 30 x 100 mm großes Stück dieses Ziegenglattleders wurde 0,1 g eines pflegenden Creme-Makeups, nämlich der Marke Nivea Beauté Teint Natur Intensive, gleichmäßig aufgebracht und für zwei bis zweieinhalb Stunden trocknen gelassen.

Für die Versuchsdurchführung wird ein kreisrundes Löschpapier mit einem Durchmesser von 57 mm (Schleicher & Schüll, Nr. 860) in der Größe des zu testenden Wattepads mit einem Durchmesser von ebenfalls 57 mm ausgestanzt und mit 0,85 - 0,9 g demineralisiertem Wasser getränkt. Das runde Wattepad (Prüfling) (Durchmesser 57 mm, 25,5 cm$^2$) wird auf das getränkte Löschpapier gelegt und 10 s lang mit 1 kg belastet. Hierbei nimmt das Wattepad etwas Flüssigkeit auf. Das oberflächlich befeuchtete Wattepad wird nun auf das Ziegenleder in einer Zugrichtung vor die "geschminkte" Stelle gelegt. Ein zylindrisches Gewicht von 300 g und Durchmesser von 57 mm mit einer Öse wird mit der Zugvorrichtung der Zugprüfmaschine mittels eines Bindfadens verbunden. Das verbundene Gewicht wird mittels Doppelklebeband in exakter Überdeckung mit dem Wattepad fixiert. Wattepad und Gewicht werden dann mit einer Geschwindigkeit von 200 mm/min über die "geschminkte" Fläche gezogen. Es werden wenigstens fünf Abschminkversuche durchgeführt und jeweils die auf dem Ziegenleder verbleibende Menge an Make-up visuell oder mittels eines Farbmessgeräts (Chromameter) beurteilt. Als Prüflinge wurden die vorstehend erörterten Wattepads PH und PH* sowie ein aus 100 Gew.-% Baumwolle bestehendes Wattepad, PH 100 % Baumwolle, verwendet, wobei letzteres unter der Marke "Labell" im Handel erhältlich ist und wasserstrahlverfestigt ist. Weiter wurde ein im Handel erhältliches Wattepad der Marke "Demak up Duo" sowie der Marke "Demak'up Supersoft" geprüft, welches ebenfalls aus 100 % Baumwolle besteht und wasserstrahlverfestigt ist. Wattepads der Marken "Nivea" und "Jean Carol" bestehen jeweils aus 100 % Baumwolle und sind ebenfalls wasserstrahlverfestigt. Ein beidseits geprägtes Wattepad der Marke "Hydra" wurde auch getestet.

Die Figuren 1 bis 8 zeigen die fotografische Aufnahme je zweier Abschminkversuche, also das Bild, welches sich ergibt, wenn, wie vorstehend beschrieben, ein Wattepad in der beschriebenen Weise einmal über die "geschminkte" Fläche gezogen wurde. Das hierfür verwandte Wattepad wurde jeweils umgekehrt an die Ausgangsstelle des Abschminkversuchs auf das Substrat aus Ziegenleder positioniert und fotografiert.

Man erkennt, dass bei dem Produkt PH und PH* eine gegenüber den Vergleichsprodukten um ein Vielfaches effektivere Reinigungswirkung oder Abschminkwirkung erzielt wurde.

Vergleicht man die erfindungsgemäßen Produkte PH und PH*, so führt die Erhöhung des Mikrofaseranteils zu einer Verbesserung der Abschminkwirkung.

Figur 9 und 10 zeigen zwei Draufsichten auf ein erfindungsgemäßes Wattepad 2 mit unterschiedlichen Prägemustern 4, welche durch Prägekalandrieren des abgelegten Wattepads erzeugt wurden. Die Rillentiefe des Prägemusters 4 beträgt wenigstens 0,4 mm. Fig. 11 zeigt Einzelheiten des Prägemusters. Es umfasst eine wabenförmige oder waffelartige Strukturierung, die durch ein zweites Prägemuster 6 in Form eines Schriftzugs 8 unterbrochen ist. Die Abstände zwischen den Wabenlinien 10 betragen zwischen 2,0 und 5 mm, insbesondere zwischen 2,5 und 4,0 mm. Das zweite Prägemuster 6 nimmt eine Fläche von bis zu 15 x 10 mm (Abmessungen c x d) ein. Der Abstand zwischen den das jeweilige zweite Prägemuster 6 aufnehmenden Bereichen beträgt 3 - 10 mm (Abmessung b - d), insbesondere 4 - 6 mm in der einen Richtung bzw. 5 - 15 mm (Abmessung a - c), insbesondere 8 - 12 mm in der anderen Richtung.

Die wabenbildenden Prägelinien 10, welche das Prägemuster 4 bilden, sind wellenförmig mit einer halben Wellenlänge g von 2 - 8 mm, insbesondere 3 - 5 mm und mit einer Höhe h der Wellenlinie von 0,5 - 2 mm, insbesondere 0,8 - 1,2 mm.

**Patentansprüche**

1. Kosmetisches Wattepad mit Faservliesmaterial bestehend aus den folgenden Faserarten, die zur Vliesbildung in einem Luftstrom vermischt und abgelegt werden, nämlich 15 - 85 Gew.-% synthetischen Mikrostapelfasern einer Länge von wenigstens 7 mm und zusätzlich bis 72 Gew.-% Baumwollfasern und gegebenenfalls wärmeschmelzbaren Bindefasern, wobei das Flächengewicht des Wattepads 60 - 250 g/m$^2$ beträgt und wobei das Wattepad derart verdichtet ist, dass es eine Höchstzugkraft in Längsrichtung (Maschinenrichtung) und in Querrichtung (quer zur Maschinenrichtung) von 5 - 30 N/25mm aufweist.

2. Kosmetisches Wattepad nach Anspruch 1, **dadurch gekennzeichnet, dass** es sich bei den Mikrostapelfasern um Polyester (PES)- oder um Viskosefasern handelt.

3. Kosmetisches Wattepad nach Anspruch 1 oder 2, **dadurch gekennzeichnet, dass** die Mikrostapelfasern an ihrer Oberfläche hydrophiliert sind.

4. Kosmetisches Wattepad nach Anspruch 1, 2 oder 3, **dadurch gekennzeichnet, dass** das Flächengewicht des Wattepads 40 - 300 g/m$^2$, insbesondere 60 - 250 g/m$^2$, insbesondere 120 - 250 g/m$^2$ und vorzugsweise 150 - 250 g/m$^2$ beträgt.

5. Kosmetisches Wattepad nach einem oder mehreren der vorstehenden Ansprüche, **dadurch gekennzeichnet, dass** der Anteil der Mikrostapelfasern 15 - 65 Gew.-% und insbesondere 20 - 30 Gew.-% beträgt.

6. Kosmetisches Wattepad nach einem oder mehreren der vorstehenden Ansprüche, **dadurch gekennzeichnet, dass** zusätzlich 15 - 65 Gew.-% und weiter insbesondere 50 - 65 Gew.-% Baumwollfasern enthalten sind.

7. Kosmetisches Wattepad nach Anspruch 6, **dadurch gekennzeichnet, dass** die Baumwollfasern Baumwollkämmlinge sind.

8. Kosmetisches Wattepad nach einem oder mehreren der vorstehenden Ansprüche, **dadurch gekennzeichnet, dass** zusätzlich wärmeschmelzbare Bindefasern enthalten sind, insbesondere in einem gewichtsprozentualen Anteil von 10 - 20 Gew.-%, insbesondere 12 - 18 Gew.-% und weiter insbesondere 12 - 15 Gew.-%.

9. Kosmetisches Wattepad nach Anspruch 8, **dadurch gekennzeichnet, dass** die Bindefasern Mehrkomponentenfasern, insbesondere Bikomponenten-Fasern sind.

10. Kosmetisches Wattepad nach Anspruch 9, **dadurch gekennzeichnet, dass** die Mehrkomponenten-Fasern eine Faserstärke von 1,3 - 10 dtex, insbesondere von 1,3 - 3,0 dtex und eine Faserlänge von 3 - 60 mm haben.

11. Kosmetisches Wattepad nach Anspruch 9 oder 10, **dadurch gekennzeichnet, dass** es sich bei den Bikomponentenfasern um Copolyester (CO-PES) / Polyester (PES)- Bikomponentenfasern handelt.

12. Kosmetisches Wattepad nach einem der Ansprüche 8 - 11, **dadurch gekennzeichnet, dass** der Schmelzpunkt der wärmeschmelzbaren Bindefasern oder der niedrig schmelzenden Komponente der Mehrkomponentenfasern geringer ist als der Schmelzpunkt der Mikrostapelfasern.

13. Kosmetisches Wattepad nach einem oder mehreren der vorstehenden Ansprüche, **dadurch gekennzeichnet, dass** die eine Seite des Wattepads oberflächenrauh ausgebildet ist, indem dort ein Schleifmittel aufgebracht ist.

14. Kosmetishes Wattepad nach Anspruch 13, **dadurch gekennzeichnet, dass** das Schleifmittel aus einem thermoplastischen Schmelzklebepulver gebildet ist, welches auf die eine Seite des Wattepads aufgesintert ist.

15. Kosmetisches Wattepad nach Anspruch 14, **dadurch gekennzeichnet, dass** das Schmelzklebepulver Polyethylen und/oder Polyamid und/oder Polyester umfasst.

16. Kosmetisches Wattepad nach Anspruch 13, 14 oder 15, **dadurch gekennzeichnet, dass** das Schleifmittel oder Schmelzklebepulver eine Körnung von 1 - 500 $\mu$m, insbesondere 1 - 100 $\mu$m, vorzuggsweise 1 - 65 $\mu$m aufweist.

17. Kosmetisches Wattepad nach einem der Ansprüche 13 - 16, **dadurch gekennzeichnet, dass** das Schleifmittel in einem Flächengewicht von 5 - 50 g/m$^2$, insbesondere 10 - 40 g/m$^2$, vorzugsweise 15 - 30 g/m$^2$ aufgebracht ist.

18. Kosmetisches Wattepad nach einem oder mehreren der vorstehenden Ansprüche, daduch gekennzeichnet, dass es herstellerseitig befeuchtet und im feuchten Zustand im wesentlichen feuchtigkeitsdicht verpackt ist.

19. Kosmetisches Wattepad nach einem oder mehreren der vorstehenden Ansprüche, **dadurch gekennzeichnet, dass** es Baumwollfasern umfasst, die zu wenigstens 0,2 Gew.-% mit einem Weichmacher beaufschlagt sind.

20. Kosmetisches Wattepad nach Anspruch 19, **dadurch gekennzeichnet, dass** der Weichmacher ein Fettsäurekondensationsprodukt und/oder funktionelle Polydimethylsiloxane und/oder Polyethylene umfasst.

21. Kosmetisches Wattepad nach einem der Ansprüche 18 - 20, **dadurch gekennzeichnet, dass** es mit einer Öl-in-Wasser-Emulsion mit einer Viskosität von < 2000mPas insbesondere von < 800 mPas befeuchtet ist.

**22.** Kosmetisches Wattepad nach Anspruch 21, **dadurch gekennzeichnet, dass** die Öl-in-Wasser-Emulsion einen Konservierungsstoff (Parabene, Benzylalkohol; 0,2 - 0,5 Gew.-%) umfasst.

**23.** Kosmetisches Wattepad nach einem oder mehreren der vorstehenden Ansprüche, **dadurch gekennzeichnet, dass** seine Lichtdurchlässigkeit weniger als 0,9 %, vorzugsweise weniger als 0,75 %, besonders bevorzugt weniger als 0,70 % und ganz besonders bevorzugt weniger als 0,6 % beträgt.

**24.** Verwendung eines Wattepads nach einem oder mehreren der vorstehenden Ansprüche zum Reinigen und/oder Abschminken der Haut.

**Claims**

**1.** Cosmetic cotton wool pad with fibre nonwoven material consisting of the following fibre types, which for nonwoven formation are mixed in an airflow and laid down, namely 15 - 85 % by weight of synthetic micro staple fibres having a length of at least 7 mm and additionally up to 72 % by weight of cotton fibres and optionally thermally meltable binding fibres, wherein the basis weight of the cotton pad is 60 - 250 g/m$^2$ and wherein the cotton pad is compressed such that it has a maximum tensile strength in a longitudinal direction (machine direction) and in a transverse direction (transverse to the machine direction) of 5 - 30 N/25 mm.

**2.** Cosmetic cotton wool pad according to claim 1, **characterised in that** the micro-staple fibres are polyester (PES) or viscose fibres.

**3.** Cosmetic cotton wool pad according to either claim 1 or claim 2, **characterised in that** the micro-staple fibres are hydrophilised on their surface.

**4.** Cosmetic cotton wool pad according to claim 1, claim 2 or claim 3, **characterised in that** the area weight of the cotton pad is 40 to 300 g/m$^2$, particular 60 to 250 g/m$^2$, in particularly 120 to 250 g/m$^2$ and preferably 150 to 250 g/m$^2$.

**5.** Cosmetic cotton wool pad according to one or more of the preceding claims, **characterised in that** the micro-staple fibre content is 15 to 65 % by weight and particularly 20 to 30 % by weight.

**6.** Cosmetic cotton wool pad according to one or more of the preceding claims, **characterised in that** 15 to 65 % by weight and more particularly 50 to 65 % by weight of cotton fibres are additionally contained.

**7.** Cosmetic cotton wool pad according to claim 6, **characterised in that** the cotton fibres are cotton noils.

**8.** Cosmetic cotton wool pad according to one or more of the preceding claims, **characterised in that** heat-meltable binding fibres are additionally contained, in particular in a percentage by weight content of 10 to 20 % by weight, particularly 12 to 18 % by weight and more particularly 12 to 15 % by weight.

**9.** Cosmetic cotton wool pad according to claim 8, **characterised in that** the binding fibres are multi-component fibres, in particular bi-component fibres.

**10.** Cosmetic cotton wool pad according to claim 9, **characterised in that** the multi-component fibres have a fibre thickness of 1.3 to 10 dtex, in particular 1.3 to 3.0 dtex and a fibre length of 3 to 60 mm.

**11.** Cosmetic cotton wool pad according to either claim 9 or claim 10, **characterised in that** the bi-component fibres are copolyester (CO PES)/polyester (PES) bi-component fibres.

**12.** Cosmetic cotton wool pad according to any one of claims 8 to 11, **characterised in that** the melting point of the heat meltable binding fibres or of the low-melting components of the multi-component fibres, is lower than the melting point of the micro-staple fibres.

**13.** Cosmetic cotton wool pad according to one or more of the preceding claims, **characterised in that** one side of the cotton wool pad has a rough surface as a result of an abrasive being applied thereto.

**14.** Cosmetic cotton wool pad according to claim 13, **characterised in that** the abrasive is formed from a thermoplastic hot-melt adhesive powder which is sintered onto one side of the cotton wool pad.

15. Cosmetic cotton wool pad according to claim 14, **characterised in that** the hot-melt adhesive powder comprises polyethylene and/or polyamide and/or polyester.

16. Cosmetic cotton wool pad according to claim 13, claim 14 or claim 15, **characterised in that** the abrasive or hot-melt adhesive powder has a grain size of 1 to 500 $\mu$m, in particular 1 to 100 $\mu$m, preferably 1 to 65 $\mu$m.

17. Cosmetic cotton wool pad according to any one of claims 13 to 16, **characterised in that** the abrasive is applied at a surface weight of 5 to 50 g/m$^2$, in particular 10 to 40 g/m$^2$, preferably 15 to 30 g/m$^2$.

18. Cosmetic cotton wool pad according to one or more of the preceding claims, **characterised in that** it is moistened by the manufacturer and is packaged in a substantially moisture-proof manner in the moist state.

19. Cosmetic cotton wool pad according to one or more of the preceding claims, **characterised in that** it comprises cotton fibres, at least 0.2 % by weight of which is acted on by a softener.

20. Cosmetic cotton wool pad according to claim 19, **characterised in that** the softener comprises a fatty acid condensation product and/or functional polydimethylsiloxanes and/or polyethylenes.

21. Cosmetic cotton wool pad according to any one of claims 18 to 20, **characterised in that** it is moistened with an oil-in-water emulsion having a viscosity of < 2,000 mPas, in particular < 800 mPas.

22. Cosmetic cotton wool pad according to claim 21, **characterised in that** the oil-in-water emulsion comprises a preservative (parabenes, benzyl alcohol; 0.2 to 0.5 % by weight).

23. Cosmetic cotton wool pad according to one or more of the preceding claims, **characterised in that** it is less than 0.9 %, preferably less than 0.75 %, particularly preferably less than 0.70 % and very particularly preferably less than 0.6 %, transparent.

24. Use of a cotton wool pad according to one or more of the preceding claims for cleansing the skin and/or removing make-up from the skin.

**Revendications**

1. Tampon d'ouate pour usage cosmétique avec matériel de fibres nontissé composé de type de fibres suivantes, les quelles ont été mélangées dans un courant d'air et déposées, à savoir 15-85% en poids de microfibres discontinues synthétiques d'une longueur d'au moins 7 mm et en supplément jusqu'à 72% en poids des fibres de coton et en particulier des fibres de liaison thermofusibles, le poids par unité de surface du tampon d'ouate étant compris entre 60-250 g/m$^2$ et le tampon d'ouate étant comprimé tel que la force de traction maximum en direction longitudinale (direction de production) et en direction transversale (transversal à la direction de production) est comprise entre 5 et 30 N/25 mm.

2. Tampon d'ouate pour usage cosmétique selon la revendication 1, **caractérisé en ce que** les microfibres discontinues sont des fibres de polyester (PES) ou de viscose.

3. Tampon d'ouate pour usage cosmétique selon la revendication 1 ou 2, **caractérisé en ce que** les microfibres discontinues sont hydrophilées sur leur surface.

4. Tampon d'ouate pour usage cosmétique selon la revendication 1, 2 ou 3, **caractérisé en ce que** le poids par unité de surface du tampon d'ouate est compris entre 40 et 300 g/m$^2$, en particulier entre 60 et 250 g/m$^2$, en particulier entre 120 et 250 g/m$^2$ et de préférence entre 150 et 250 g/m$^2$.

5. Tampon d'ouate pour usage cosmétique selon l'une ou plusieurs des revendications précédentes, **caractérisé en ce que** la part des microfibres discontinues est comprise entre 15 et 65 % en poids et en particulier entre 20 et 30 % en poids.

**6.** Tampon d'ouate pour usage cosmétique selon l'une ou plusieurs des revendications précédentes, **caractérisé en ce que** des fibres de coton sont contenues en supplément représentant de 15 à 65 % en poids et plus particulièrement de 50 à 65 % en poids.

**7.** Tampon d'ouate pour usage cosmétique selon la revendication 6, **caractérisé en ce que** les fibres de coton sont des débourrures de coton.

**8.** Tampon d'ouate pour usage cosmétique selon l'une ou plusieurs des revendications précédentes, **caractérisé en ce que** des fibres de liaison thermofusibles sont contenues en supplément, en particulier avec un pourcentage en poids compris entre 10 et 20 % en poids, en particulier entre 12 et 18 % en poids et plus particulièrement entre 12 et 15 % en poids.

**9.** Tampon d'ouate pour usage cosmétique selon la revendication 8, **caractérisé en ce que** les fibres de liaison sont des fibres à plusieurs composants, en particulier des fibres à deux composants.

**10.** Tampon d'ouate pour usage cosmétique selon la revendication 9, **caractérisé en ce que** les fibres à plusieurs composants ont une épaisseur de fibres comprise entre 1,3 et 10 dtex, en particulier entre 1,3 et 3 dtex et une longueur de fibres comprise entre 3 et 60 mm.

**11.** Tampon d'ouate pour usage cosmétique selon la revendication 9 ou 10, **caractérisé en ce que** les fibres à deux composants sont des fibres à deux composants copolyester (CO-PES) / polyester (PES).

**12.** Tampon d'ouate pour usage cosmétique selon l'une des revendications 8 à 11, **caractérisé en ce que** le point de fusion des fibres de liaison thermofusibles ou du composant des fibres à plusieurs composants ayant le point de fusion le plus bas est inférieur au point de fusion des microfibres discontinues.

**13.** Tampon d'ouate pour usage cosmétique selon l'une ou plusieurs des revendications précédentes, **caractérisé en ce qu'**une face du tampon d'ouate présente une rugosité de surface par dépôt d'un abrasif.

**14.** Tampon d'ouate pour usage cosmétique selon la revendication 13, **caractérisé en ce que** l'abrasif est formé d'une poudre thermoplastique adhésive par fusion qui est agglomérée par frittage sur une face du tampon d'ouate.

**15.** Tampon d'ouate pour usage cosmétique selon la revendication 14, **caractérisé en ce que** la poudre adhésive par fusion comprend du polyéthylène et/ou du polyamide et/ou du polyester.

**16.** Tampon d'ouate pour usage cosmétique selon la revendication 13, 14 ou 15, **caractérisé en ce que** l'abrasif ou la poudre adhésive par fusion présente une granulométrie comprise entre 1 et 500 $\mu$m, en particulier entre 1 et 100 $\mu$m, de préférence entre 1 et 65 $\mu$m.

**17.** Tampon d'ouate pour usage cosmétique selon l'une des revendications 13 à 16, **caractérisé en ce que** l'abrasif déposé a un poids par unité de surface compris entre 5 et 50 g/m$^2$, en particulier entre 10 et 40 g/m$^2$, de préférence entre 15 et 30 g/m$^2$.

**18.** Tampon d'ouate pour usage cosmétique selon l'une ou plusieurs des revendications précédentes, **caractérisé en ce qu'**il est humidifié par le fabricant et emballé à l'état humide de manière essentiellement étanche à l'humidité.

**19.** Tampon d'ouate pour usage cosmétique selon l'une ou plusieurs des revendications précédentes, **caractérisé en ce qu'**il comprend des fibres de coton qui contiennent un assouplissant pour au moins 0,2 % en poids.

**20.** Tampon d'ouate pour usage cosmétique selon la revendication 19, **caractérisé en ce que** l'assouplissant comprend un produit de condensation d'acide gras et/ou des polydiméthysiloxanes fonctionnels et/ou des polyéthylènes.

**21.** Tampon d'ouate pour usage cosmétique selon l'une des revendications précédentes 18 à 20, **caractérisé en ce qu'**il est humidifié avec une émulsion oléoaqueuse de viscosité inférieure à 2000 mPas, en particulier inférieure à 800 mPas.

**22.** Tampon d'ouate pour usage cosmétique selon la revendication 21, **caractérisé en ce que** l'émulsion oléoaqueuse comprend une substance de conservation (parahydroxybenzoates, alcool benzylique ; 0,2 à 0,5 % en poids).

**23.** Tampon d'ouate pour usage cosmétique selon l'une ou plusieurs des revendications précédentes, **caractérisé en ce que** sa transparence est inférieure à 0,9 %, de préférence inférieure à 0,75 %, en particulier de préférence inférieure à 0,70 % et, de façon particulièrement préférée, inférieure à 0,6 %.

**24.** Utilisation d'un tampon d'ouate selon l'une ou plusieurs des revendications précédentes pour nettoyer et/ou démaquiller la peau.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1106723 A
- EP 0836842 B1
- EP 0405043 B1
- WO 0076384 A1
- US 4100324 A
- EP 1106723 A2
- WO 9925318 A1